(19) **Europäisches Patentamt**
**European Patent Office**
**Office européen des brevets**

(11) **EP 1 775 342 A1**

(12) **EUROPEAN PATENT APPLICATION**
published in accordance with Art. 158(3) EPC

(43) Date of publication:
**18.04.2007 Bulletin 2007/16**

(51) Int Cl.:
*C12N 5/10* (2006.01)   *A61K 35/12* (2006.01)
*A61K 48/00* (2006.01)   *A61P 31/00* (2006.01)
*A61P 35/00* (2006.01)   *C12N 15/09* (2006.01)

(21) Application number: **05736740.1**

(22) Date of filing: **28.04.2005**

(86) International application number:
**PCT/JP2005/008124**

(87) International publication number:
**WO 2006/001120 (05.01.2006 Gazette 2006/01)**

(84) Designated Contracting States:
**AT BE BG CH CY CZ DE DK EE ES FI FR GB GR
HU IE IS IT LI LT LU MC NL PL PT RO SE SI SK TR**

(30) Priority: **24.06.2004 JP 2004187028
29.10.2004 JP 2004016089**

(71) Applicant: **DNAVEC Research, Inc.
Tsukuba-shi,
Ibaraki 305-0856 (JP)**

(72) Inventors:
• **OKANO, Shinji,
15-3-301, Yoshizuka 4-chome,
Fukuoka-shi,
Fukuoka 8120041 (JP)**
• **YONEMITSU, Yoshikazu
8130043 (JP)**

• **SUEISHI, Katsuo
8150073 (JP)**
• **SHIBATA, Satoko
Fukuoka-shi,
Fukuoka 8120061 (JP)**
• **HASEGAWA, Mamoru,
c/o DNAVEC RESEARCH INC.,
Tsukuba-shi,
Ibaraki 3050856 (JP)**
• **KONDO, Haruhiko,
4-1303, Urbane Rivie Shimizu,
Fukuoka-shi,
Fukuoka 8150031 (JP)**

(74) Representative: **Vossius & Partner
Siebertstrasse 4
81675 München (DE)**

(54) **ANTICANCER AGENT CONTAINING MINUS-STRAND RNA VIRUS**

(57)     Minus-strand RNA viruses were found to have an effective tumor-suppressive effect even when they did not carry a therapeutically effective gene. The present invention provides anticancer agents comprising a minus-strand RNA virus. Furthermore, the present invention provides methods for producing the anticancer agents, which comprise the step of mixing a minus-strand RNA virus with pharmaceutically acceptable carriers. Furthermore, the present invention provides methods for suppressing cancer, which comprise the step of administering a minus-strand RNA virus to cancer tissues.

EP 1 775 342 A1

# EP 1 775 342 A1

**Description**

<u>Technical Field</u>

The present invention relates to the field of cancer therapy.

<u>Background Art</u>

In recent years, clinical studies have been carried out on virotherapy that targets advanced cancer and uses replicative viruses. Virotherapy is a therapy that infects tumor cells with replicative viruses, such as HSV-1 or adenovirus, and uses the viral cytocidal effect that accompanies viral propagation for treating tumor. In the case of HSV-1 or adenovirus, the replicative viruses for tumor therapy are mutant viruses whose viral genome has been genetically engineered, such that while their ability to replicate in tumors is maintained, their pathogenicity in normal human tissues is suppressed to a minimum. Therapeutic replicative viruses infect tumor cells and replicate inside the cells, and the infected cells die out in this process. The propagated viruses reinfect the surrounding tumor cells, and spread the antitumor effect (Alemany R. et al., Replicative adenoviruses for cancer therapy. Nat. Biotechnol., 2000, 18:723-727; Curiel, D.T., The development of conditionally replicative adenoviruses for cancer therapy, Clin. Cancer Res., 2000, 6:3395-9; Kirn, D., Virotherapy for cancer: Current status, hurdles, and future directions., Cancer Gene Therapy, 9:959-960, 2002; Mineta T. et al., Attenuated multi-mutated herpes simplex virus-1 for the treatment of malignant gliomas. Nat. Med. 1:938-943, 1995). Anticancer virotherapy has a wide range of applications and provides excellent practicality: for example, it can be used in combination with conventional therapy such as surgery, radiation therapy, and chemotherapy; it can be applied to solid cancers in general; it can be administered repeatedly; and it can be made to directly incorporate into the viral genome therapeutic genes such as those of cytokines to enhance antitumor effect. Development of a more effective virotherapy is expected to contribute greatly to cancer therapy.

Non-Patent Document 1: Alemany R. et al., Replicative adenoviruses for cancer therapy. Nat Biotechnol., 2000, 18: 723-727.

Non-Patent Document 2: Curiel, D.T., The development of conditionally replicative adenoviruses for cancer therapy., Clin Cancer Res., 2000, 6:3395-9.

Non-Patent Document 3: Kirn, D., Virotherapy for cancer: Current status, hurdles, and future directions, Cancer Gene Therapy, 2002, 9:959-960.

Non-Patent Document 4: Mineta T. et al., Attenuated multi-mutated herpes simplex virus-1 for the treatment of malignant gliomas. Nat Med, 1995, 1:938-943.

<u>Disclosure of the Invention</u>

<u>Problems to be Solved by the Invention</u>

An objective of the present invention is to provide a more effective anticancer virotherapy and anticancer agents. More specifically, the present invention provides methods for producing anticancer agents comprising a minus-strand RNA virus, and cancer therapeutic methods using a minus-strand RNA virus.

<u>Means to Solve the Problems</u>

To verify the therapeutic effect of *in vivo* administration of RNA virus on tumor, the present inventors performed experiments using mice subjected to ventral subcutaneous inoculation with melanoma cell lines. As a result, the present inventors found that tumor size is significantly reduced when a minus-strand RNA virus that does not carry any therapeutic gene is injected into the tumor of the animals. Therefore, this revealed that *in vivo* administration of a minus-strand RNA virus exhibits antitumor effects even when the virus does not carry any therapeutic gene.

Minus-strand RNA viruses are considered to be effective anticancer agents themselves. Moreover, direct injection of such viruses into tumor sites (cancer tissues) is expected to yield effective antitumor effects, and such viruses are useful for treating cancer.

Therefore, the present invention relates to antitumor agents comprising a minus-strand RNA virus, methods for producing the antitumor agents, and methods for suppressing cancer using the RNA virus, and more specifically, it relates to inventions described in each claim. An invention or a combination of inventions set forth in a claim(s) citing the same claim is already included in inventions described in the claims. More specifically, the present invention relates to:

[1] an anticancer agent comprising a minus-strand RNA virus;
[2] the anticancer agent of [1], wherein the minus-strand RNA virus does not encode a foreign protein;

[3] the anticancer agent of [1] or [2], wherein the minus-strand RNA virus is an infectious or a non-infectious virion;

[4] the anticancer agent of any one of [1] to [3], wherein the RNA virus is a genomic RNA-protein complex;

[5] the anticancer agent of any one of [1] to [4], wherein the minus-strand RNA virus is a paramyxovirus;

[6] the anticancer agent of [5], wherein the paramyxovirus is a Sendai virus;

[7] a method for producing an anticancer agent, comprising the step of mixing a minus-strand RNA virus with a pharmaceutically acceptable carrier or medium; and

[8] a method for suppressing cancer, comprising the step of administering a minus-strand RNA virus to a cancer tissue.

Brief Description of the Drawings

Fig. 1 depicts the therapeutic effects of *in vivo* administration of a GFP-expressing SeV, a soluble FGF receptor-expressing SeV, or a soluble PDGFR$\alpha$-expressing SeV on melanoma.

Fig. 2 depicts the growth curve of B16 melanoma cells that were inoculated subcutaneously.

Fig. 3 depicts the results of $^{51}$Cr release assay for YAC-1 target cells.

Fig. 4 depicts the results of $^{51}$Cr release assay for TRP2 peptide + EL-4.

Best Mode for Carrying Out the Invention

The present invention provides anticancer agents (carcinostatic agents) comprising a minus-strand RNA virus as an active component. In the present invention, an RNA virus refers to a virus carrying an RNA genome.

The RNA virus that works as an active component of the anticancer agents of the present invention is preferably a minus-strand RNA virus. A minus-strand RNA virus refers to viruses that include a minus strand (an antisense strand corresponding to a sense strand encoding viral proteins) RNA as the genome. The minus-strand RNA is also referred to as negative strand RNA. The minus-strand RNA virus used in the present invention particularly includes single-stranded minus-strand RNA viruses (also referred to as non-segmented minus-strand RNA viruses). The "single-strand negative strand RNA virus" refers to viruses having a single-stranded negative strand [*i.e.*, a minus strand] RNA as the genome. Such viruses include viruses belonging to Paramyxoviridae (including the genera *Paramyxovirus, Morbillivirus, Rubulavirus,* and *Pneumovirus*), Rhabdoviridae (including the genera *Vesiculovirus, Lyssavirus,* and *Ephemerovirus*), Filoviridae, Orthomyxoviridae, (including Influenza viruses A, B, and C, and Thogoto-like viruses), Bunyaviridae (including the genera *Bunyavirus, Hantavirus, Nairovirus,* and *Phlebovirus*), Arenaviridae, and the like.

A minus-strand RNA virus preferably used in the context of the present invention includes, for example, Sendai virus belonging to Paramyxoviridae. Other examples include Newcastle disease virus, mumps virus, measles virus, respiratory syncytial virus (RS virus), rinderpest virus, distemper virus, simian parainfluenza virus (SV5), and human parainfluenza viruses 1, 2, and 3; influenza virus belonging to Orthomyxoviridae; and vesicular stomatitis virus and rabies virus belonging to Rhabdoviridae.

Further examples of virus that may be used in the context of the present invention include: Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). A more preferred example is a virus selected from the group consisting of Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), and Nipah virus (Nipah).

More preferably, viruses of the present invention are preferably those belonging to Paramyxoviridae (including *Respirovirus, Rubulavirus,* and *Morbillivirus*) or derivatives thereof, and more preferably those belonging to the genus *Respirovirus* (also referred to as *Paramyxovirus*) or derivatives thereof. The derivatives include viruses that are genetically-modified or chemically-modified in a manner not to impair their gene-transferring ability. Examples of viruses of the genus *Respirovirus* applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10).

The minus-strand RNA viruses of the present invention are most preferably Sendai viruses.

The minus-strand RNA viruses of the present invention may be derived from natural strains, wild-type strains, mutant strains, laboratory-passaged strains, artificially constructed strains, or such. More specifically, the minus-strand RNA viruses may be minus-strand RNA viruses isolated from nature, or minus-strand RNA viruses artificially generated by genetic recombination. Furthermore, as long as the ability to replicate genomic RNA in infected cells is maintained, any of the genes carried by wild-type minus-strand RNA viruses can have mutations or deletions. For example, minus-strand RNA viruses comprising a mutation or deletion in at least an envelope protein or coat protein-encoding gene of the minus-strand RNA viruses can be preferably used. Such minus strand RNA viruses can replicate the RNA genome in infected cells but cannot form infectious virions. Since there is no need to be concerned about spread of infection to the surrounding, the viruses are very safe. For example, a minus-strand RNA virus that does not comprise at least one or a combination of the genes encoding spike proteins or envelope proteins such as F, H, HN, or G can be used (WO00/70055 and WO00/70070; Li, H.-O. et al., J. Virol. 74(14) 6564-6569 (2000)). A genome can be replicated in the infected cells if proteins that are necessary for genome replication (for example N, P, and L proteins) are encoded in the genomic RNAs. Defective-type viruses can be produced, for example, by externally supplying to virus-producing cells, products of the deficient genes or proteins which can complement the deficient genes (WO00/70055 and WO00/70070; Li, H.-O. et al., J. Virol. 74(14) 6564-6569 (2000)). However, for example, among minus-strand RNA viruses, viruses carrying the M protein gene release non-infectious virions (VLP) even if they do not carry genes encoding spike proteins such as F protein or HN protein; therefore, VLP can be produced without complementing the viral proteins (WO00/70070). Furthermore, RNP comprising genomic RNA and the N, L, and P proteins can be amplified in cells even if they do not have any envelope protein genes; therefore, RNP can be collected from cell lysates by centrifugation and such.

Moreover, the anticancer agents of the present invention can be produced using mutant RNA viruses. For example, many temperature-sensitive mutations of envelope proteins and coat proteins are known. RNA viruses comprising these temperature-sensitive mutant protein genes can be preferably used in the present invention. Temperature-sensitive mutations refer to mutations that significantly decrease protein activity at normal temperatures (for example, 37°C to 38°C) of the virus' host, as compared with the activity at low temperatures (for example, 30°C to 32°C). Such proteins harboring temperature-sensitive mutations are useful because they enable virus production at permissible temperatures (low temperatures).

Examples of temperature-sensitive mutations of the M gene of minus-strand RNA viruses include amino acid substitutions at a site arbitrarily selected from the group consisting of G69, T116, and A183 in the Sendai virus M protein (Inoue, M. et al., J. Virol. 2003, 77: 3238-3246). The amino acids of the homologous sites in the M protein of other minus-strand RNA viruses can be identified easily, and specifically, examples of homologous sites in M proteins that correspond to G69 of the SeV M protein include (the letter and number denote the amino acid and its position; abbreviation of the names is shown inside the parentheses): G69 in human parainfluenza virus-1 (HPIV-1); G73 in human parainfluenza virus-3 (HPIV-3); G70 in phocine distemper virus (PDV) and canine distemper virus (CDV); G71 in dolphin molbillivirus (DMV); G70 in peste-des-petits-ruminants virus (PDPR), measles virus (MV), and rinderpest virus (RPV); G81 in Hendra virus (Hendra) and Nipah virus (Nipah); G70 in human parainfluenza virus-2 (HPIV-2); E47 in human parainfluenza virus-4a (HPIV-4a) and human parainfluenza virus-4b (HPIV-4b); and E72 in mumps virus (Mumps). Examples of M protein homologous sites that correspond to T116 in the SeV M protein include: T116 in human parainfluenza virus-1 (HPIV-1); T120 in human parainfluenza virus-3 (HPIV-3); T104 in phocine distemper virus (PDV) and canine distemper virus (CDV); T105 in dolphin molbillivirus (DMV); T104 in peste-des-petits-ruminants virus (PDPR), measles virus (MV), and rinderpest virus (RPV); T120 in Hendra virus (Hendra) and Nipah virus (Nipah); T117 in human parainfluenza virus-2 (HPIV-2) and simian parainfluenza virus 5 (SV5); T121 in human parainfluenza virus-4a (HPIV-4a) and human parainfluenza virus-4b (HPIV-4b); T119 in mumps virus (Mumps); and S120 in Newcastle disease virus (NDV). Examples af M protein homologous sites that correspond to A183 in the SeV M protein include: A183 in human parainfluenza virus-1 (HPIV-1); F187 in human parainfluenza virus-3 (HPIV-3); Y171 in phocine distemper virus (PDV) and canine distemper virus (CDV); Y172 in dolphin molbillivirus (DMV); Y171 in peste-des-petits-ruminants virus (PDPR), measles virus (MV), and rinderpest virus (RPV); Y187 in Hendra virus (Hendra) and Nipah virus (Nipah); Y184 in human parainfluenza virus-2 (HPIV-2); F184 in simian parainfluenza virus 5 (SV5); F188 in human parainfluenza virus-4a (HPIV-4a) and human parainfluenza virus-4b (HPIV-4b); F186 in mumps virus (Mumps); and Y187 in Newcastle disease virus (NDV). Among the viruses recited herein, viruses comprising a genome encoding, as their M protein, a mutant M protein in which the amino acids of any one of the three sites, preferably a combination of any two of these sites. More preferably, all three sites mentioned above are substituted with other amino acids are used in the present invention.

A preferred amino acid mutation is substitution to another amino acid whose side chains have different chemical properties. For example, substitution is carried out with an amino acid whose BLOSUM62 matrix value (Henikoff, S. and Henikoff, J. G. (1992) Proc. Natl. Acad. Sci. USA 89: 10915-10919) is 3 or less, preferably 2 or less, more preferably 1 or less, and even more preferably 0 or less. Specifically, G69, T116, and A183 of the Sendai virus M protein or homologous sites of other viral M proteins can be substituted with Glu (E), Ala (A), and Ser (S), respectively. It is also possible to use mutations that are homologous to the mutations in the M protein of a temperature-sensitive P253-505 measles virus strain (Morikawa, Y. et al., Kitasato Arch. Exp. Med. 1991: 64; 15-30). Mutations can be introduced, for example, by using oligonucleotides and such according to well known methods for introducing mutations.

Examples of temperature-sensitive mutations of the HN gene include amino acid substitutions at any of the sites selected from the group consisting of A262, G264, and K461 in the Sendai virus HN protein (Inoue, M. et al., J.

Virol. 2003, 77: 3238-3246). In a preferred example, A262, G264, and K461 in the Sendai virus HN protein or homologous sites of other viral HN proteins are substituted with Thr (T), Arg (R), and Gly (G), respectively. Furthermore, for example, the amino acids at positions 464 and 468 in the HN protein can be mutated by referring to the temperature-sensitive mumps virus vaccine strain, Urabe AM9 (Wright, K. E. et al., Virus Res. 2000: 67; 49-57).

Minus-strand RNA viruses may comprise mutations in the P gene or L gene. Specific examples of such mutations include mutation of Glu at position 86 (E86) of the SeV P protein, substitution of Leu at position 511 (L511) of the SeV P protein to another amino acid, or substitution of homologous sites in the P protein of a different minus-strand RNA virus. Specific examples include substitution of the amino acid at position 86 to Lys, and substitution of the amino acid at position 511 to Phe. Regarding the L protein, examples include substitution of Asn at position 1197 (N1197) and/or Lys at position 1795 (K1795) in the SeV L protein to other amino acids, or substitution of homologous sites in the L protein of another minus-strand RNA virus, and specific examples include substitution of the amino acid at position 1197 to Ser, and substitution of the amino acid at 1795 to Glu. Mutations of the P gene and L gene can significantly increase the effects of persistent infectivity, suppression of the release of secondary virions, and suppression of cytotoxicity. Furthermore, by combining mutation and/or deletion of the envelope protein gene, these effects can be increased dramatically.

When using enveloped viruses, it is possible to use viruses that comprise in their envelope, proteins that are different from the envelope proteins originally carried by the viruses. For example, by expressing a desired foreign envelope protein in the virus-producing cells during virus production, viruses comprising such protein can be produced. Such protein is not particularly limited, and desired proteins that confer the ability to infect mammalian cells are used. A specific example is the vesicular stomatitis virus (VSV) G protein (VSV-G). The VSV-G protein may be derived from any VSV strain, and for example, VSV-G protein derived from the Indiana serotype strain (J. Virology 39: 519-528 (1981)) can be used, but it is not limited thereto. The minus-strand RNA viruses used in the present invention can comprise an arbitrary combination of envelope proteins derived from other viruses.

A characteristic of the minus-strand RNA viruses of the present invention is that they themselves have anticancer effects. Specifically, the minus-strand RNA viruses do not need to comprise foreign proteins or nucleic acids encoding the proteins. Therefore, the present invention relates to anticancer agents comprising minus-strand RNA viruses that do not encode foreign proteins. The minus-strand RNA viruses of the present invention may or may not encode a foreign protein or a foreign gene in their genomic RNA. Since minus-strand RNA viruses that do not encode any foreign proteins also exhibit anticancer (carcinostatic) effects, foreign genes are not necessarily required. Therefore, the present invention is advantageous in that a desired minus-strand RNA virus such as wild-type minus-strand RNA virus, or minus-strand RNA virus isolated from nature (including mutant strains) can be used. For example, RNA viruses used in the present invention may be RNA viruses that do not encode proteins with anticancer therapeutic effects. Such viruses include RNA viruses encoding desired foreign proteins that do not have any anticancer therapeutic effects. For example, RNA viruses encoding marker proteins such as green fluorescent protein (GFP), luciferase, or various peptide tags can be used to detect the introduction of RNA viruses. Alternatively, by further incorporating into the minus-strand RNA viruses foreign proteins or foreign genes that assist anticancer (carcinostatic) effects, the carcinostatic (anticancer) effects can also be further enhanced.

Recombinant minus-strand RNA viruses that carry foreign genes can be reconstituted using well-known methods. Specific procedures for such production typically include the steps of (a) transcribing a cDNA encoding the genomic RNA of a minus-strand RNA virus in a cell expressing viral proteins necessary for virion formation, and (b) collecting the culture supernatant comprising the produced virus. The viral proteins can be expressed from the transcribed viral genomic RNA, or they may be provided in trans from sources other than the genomic RNA. When viral genes that are required for virion formation are deficient from the genomic RNA, these viral genes are separately expressed in the virus-producing cells to complement virion formation. To express a viral protein or RNA genome in cells, a vector, in which a DNA encoding the protein or the genomic RNA is linked downstream of a suitable promoter that functions in the host cells, is introduced into the host cells. Transcribed genomic RNAs are replicated in the presence of viral proteins, and infectious virions are formed. When producing deficient viruses that lack envelope protein genes and such, the deficient proteins, viral proteins that can complement their functions, or such are expressed in the virus-producing cells.

For example, the minus-strand RNA viruses of the present invention can be produced using the following known methods (WO97/16539; WO97/16538 WO00/70055; WO00/70070; WO01/18223; Hasan, M. K. et al., J. Gen. Virol. 78: 2813-2824, 1997; Kato, A. et al., 1997, EMBO J. 16: 578-587; Yu, D. et al., 1997, Genes Cells 2: 457-466; Durbin, A. P, et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995. EMBO J. 14: 6087-6094; Kato, A. et al., 1996, Genes Cells 1: 569-579; Baron, M. D. and Barrett, T., 1997, J. Virol. 71: 1265-1271; and Bridgen, A. and Elliott, R. M., 1996, Proc. Natl. Acad. Sci. USA 93: 15400-15404). Using these methods, minus-strand RNA viruses comprising parainfluenza virus, vesicular stomatitis virus, rabies virus, measles virus, rinderpest virus, and Sendai virus can be reconstituted from DNA. In the present invention, it is preferable to use minus-strand RNA viruses, especially single-stranded minus-strand RNA viruses, more preferably viruses of the *Paramyxoviridae* family, and even more preferably viruses of the genus *Respirovirus.*

There is no particular limitation on the foreign genes to be carried by the minus-strand RNA viruses, and examples of naturally occurring proteins include hormones, cytokines, growth factors, receptors, intracellular signaling molecules, enzymes, antibodies (including full-length antibodies, antibody fragments such as Fab, single-chain antibodies, etc), and peptides. The proteins may be secretory proteins, membrane proteins, cytoplasmic proteins, nuclear proteins, and such. Artificial proteins include, for example, fusion proteins of chimeric toxins and such, dominant negative proteins (including soluble receptor molecules or membrane-bound dominant negative receptors), cell surface molecules and truncated cell adhesion molecules. The proteins may also be proteins to which a secretory signal, membrane-localization signal, nuclear translocation signal, or such has been attached. The function of a particular gene can be suppressed by expressing antisense RNA molecules, RNA-cleaving ribozymes, or the like as transgenes. Carcinostatic effects may be enhanced by preparing viruses using a therapeutic gene showing carcinostatic effects as a foreign gene.

For example, the use of a gene that inhibits vascularization or angiogenesis can further enhance antitumor effects. Examples of genes that are known to promote vascularization or angiogenesis include fibroblast growth factor 2 (FGF2) (Baffour, R. et al., J. Vasc. Surg. 16(2):181-91, 1992), endothelial cell growth factor (ECGF) (Pu, L. Q. et al., J. Surg. Res. 54(6):575-83, 1993), vascular endothelial growth factor (VEGF)/vascular permeability factor (VPF) (Takeshita, S. et al., Circulation 90 (5 Pt 2):II228-34, 1994; Takeshita, S. et al., J. Clin, Invest. 93(2):662-70, 1994), and hepatocyte growth factor/scatter factor (HGF/SF). Genes that encode secretory proteins which inhibit the effect of these signal molecules can be used as foreign genes. Specific examples include antibodies that bind to these signal molecules or their receptors, or polypeptides comprising antigen-binding fragments of such antibodies, or soluble forms of such receptor proteins (secretory receptors that carry a ligand binding site, but not transmembrane region). In particular, minus-strand RNA viruses encoding soluble-type FGF receptor (FGF-R) polypeptides can significantly increase the effects of cancer growth suppression. Therefore, minus-strand RNA viruses that encode soluble FGF-R can be preferably used in the present invention. For the soluble FGF-R, naturally occurring soluble FGF-R may be used, or fragments comprising an extracellular domain of membrane-bound FGF-R (FGF-R1 and such) may be used (A. Hanneken and A. Baird, Investigative Ophthalmology & Visual Science, Vol 36, 1192-1196, 1995; Takaishi, S. et al., Biochem. Biophys. Res. Commun., 267(2):658-62, 2000; Seno M, et al., Cytokine, 10(4):290-4, 1998; and Hanneken, A., FEBS Lett. 489: 176, 2001).

Furthermore, anticancer immune response is increased when the immune system is stimulated upon expression of cytokines; therefore, minus-strand RNA viruses into which cytokine-encoding genes have been introduced are also useful. A minus-strand RNA virus carrying a gene encoding an immunostimulatory cytokine will serve as an effective anticancer agent having the activity to induce tumor immunity. For example, immunostimulatory cytokines comprise interleukins (for example, IL-1alpha, IL-1beta, IL-2, IL-3, IL-4, IL-6, IL-7, IL-8, IL-9, IL-10, IL-12, IL-15, IL-18, IL-19, IL-20, IL-21, IL-23, and IL-27), interferons (for example, IFN-alpha, IFN-beta, and IFN-gamma), tumor necrosis factor (TNF), transforming growth factor (TGF)-beta, granulocyte colony stimulating factor (G-CSF), macrophage colony stimulating factor (M-CSF), granulocyte macrophage colony stimulating factor (GM-CSF), insulin-like growth factor (IGF)-I, IGF-2, Flt-3 ligand, Fas ligand, c-kit ligand, and other immunomodulatory proteins (such as chemokines and costimulatory molecules).

The amino acid sequences of these cytokines are well known to those skilled in the art. One may refer to: for IL-4, for example, Arai et al. (1989), J. Immunol. 142(1) 274-282; for IL-6, for example, Yasukawa et al. (1987), EMBO J., 6(10): 2939-2945; for IL-12, for example, Wolf et al. (1991), J. Immunol. 146(9): 3074-3081; for IFN-alpha, for example, Gren et al. (1984) J. Interferon Res. 4(4): 609-617, and Weismann et al. (1982) Princess Takamatsu Symp. 12: 1-22. Examples of IFN-beta sequences include the sequence from position 139 to 636 of Accession number NM_002176 (corresponding to position 22 to 187 of the amino acid sequence of NP_002167) (Derynck, R. et al., Nature 285, 542-547 (1980); Higashi, Y et al., J. Bio,. Chem. 258, 9522-9529 (1983); Kuga, T. et al., Nucleic Acid Res. 17, 3291 (1989). Moreover, one may refer to: for TNF, for example, Pennica et al. (1984) Nature 312: 724-729; for G-CSF, for example, Hirano et al. (1986) Nature 324:73-76; and for GM-CSF, for example, Cantrell et al. (1985) Proc. Natl. Acad. Sci. (USA) 82(18): 6250-6254. More specifically, the nucleic acid sequence encoding GM-CSF includes sequences containing the sequences from position 84 to 461 of Accession number NM_000758 8 (corresponding to position 18 to 144 of the amino acid sequence ofNP_000749). The nucleic acid sequence encoding IL-4 includes sequences containing the sequences from position 443 to 829 of Accession number NM_000589 (corresponding to position 25 to 153 of the amino acid sequence of NP_000580). Signal peptides can be appropriately substituted with signal peptide sequences of other proteins. Using natural genes encoding these cytokines or using the degeneracy of the genetic code, mutant genes encoding functional cytokines can be constructed and used.

Moreover, genes may be modified to express modified forms of the cytokines. For example, a cytokine that has two forms, the precursor form and mature form (for example, cytokines producing active fragments by cleavage of their signal peptides or by limited proteolysis) may be genetically modified to express either one of the precursor form and the mature form. Other modified forms, for example, fusion proteins formed between an active fragment of a cytokine and a heterologous sequence (for example, a heterologous signal peptide) can also be used.

In the present invention, "recombinant virus" refers to a virus produced via a recombinant polynucleotide or to an amplification product of a virus. "Recombinant polynucleotide" refers to a polynucleotide in which one or both ends are not linked as in the natural condition. Specifically, a recombinant polynucleotide is a polynucleotide in which the linkage of the polynucleotide chain has been artificially modified (cleaved and/or linked). Recombinant polynucleotides can be produced using well known gene recombination methods by combining polynucleotide synthesis, nuclease treatment, ligase treatment, and such. Recombinant viruses can be produced by expressing a genetically engineered polynucleotide encoding a viral genome, and then reconstituting the virus. For example, methods for reconstructing a virus from cDNA that encodes the viral genome are known (Y. Nagai, A. Kato, Microbiol. Immunol., 43, 613-624 (1999)).

In the present invention, "gene" refers to a genetic substance, a nucleic acid having a sequence to be transcribed in a sense or antisense strand. Genes may be RNAs or DNAs. In this invention, a nucleic acid encoding a protein is referred to as a gene of that protein. Further, a gene may not encode a protein. For example, a gene may encode a functional RNA, such as a ribozyme or antisense RNA. A gene may be a naturally-occurring or artificially designed sequence. Furthermore, in the present invention, "DNA" includes both single-stranded and double-stranded DNAs. Moreover, "encoding a protein" means that a polynucleotide includes an ORF that encodes an amino acid sequence of the protein in a sense or antisense strand, so that the protein can be expressed under appropriate conditions.

Minus-strand RNA viruses may encode an antisense strand for a foreign gene in the genomic RNA as required. Genomic RNA refers to RNA that has the function to form a ribonucleoprotein (RNP) with the viral proteins of a minus-strand RNA virus. Genes contained in the genome are expressed by the RNP, genomic RNA is replicated, and daughter RNPs are formed. In general, the genome of a minus-strand RNA virus is constituted so that the viral genes are situated in an antisense orientation between the 3'-leader region and 5'-trailer region. Between the ORFs of individual genes exists a transcription ending sequence (E sequence)-intervening sequence (I sequence)- transcription starting sequence (S sequence) that allows the RNA encoding each ORF to be transcribed as a separate cistron.

Genes encoding the viral proteins of a minus-strand RNA virus include NP, P, M, F, HN, and L genes. "NP, P, M, F, HN, and L genes" refer to genes encoding nucleocapside-, phospho-, matrix-, fusion-, hemagglutinin-neuraminidase-, and large-proteins respectively. Genes in each virus belonging to Paramyxovirinae are commonly listed as follows. In general, NP gene is also listed as "N gene."

| | | | | | | | |
|---|---|---|---|---|---|---|---|
| *Respirovirus* | NP | P/C/V | M | F | HN | - | L |
| *Rubulavirus* | NP | P/V | M | F | HN | (SH) | L |
| *Morbillivirus* | NP | P/C/V | M | F | H | - | L |

For example, the database accession numbers for the nucleotide sequences of each of the Sendai virus genes are: M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30242, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Examples of viral genes encoded by other viruses are: CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for P gene; CDV, AF014953; DMV, Z47758; HPIV-1, M74081; HPIV-3, D00047; MV, ABO16162; RPV, X68311; SeV, AB005796; and Tupaia, AF079780 for C gene; CDV, M12669; DMV, Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for M gene; CDV, M21849; DMV, AJ224704; HPN-1, M22347; HPIV-2, M60182; HPIV-3, X05303; HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for F gene; and, CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, Z81358; PDV, Z36979; RPV, AF132934; SeV, U06433; and SV-5, S76876 for HN (H or G) gene. However, a number of strains are known for each virus, and genes exist that include sequences other than those cited above, due to strain variation.

The ORFs encoding these viral proteins and ORFs of the foreign genes are arranged in the antisense direction in the genomic RNAs, via the above-described E-I-S sequence. The ORF closest to the 3'-end of the genomic RNAs requires only an S sequence between the 3'-leader region and the ORF, and does not require an E or I sequence. Further, the ORF closest to the 5'-end of the genomic RNA requires only an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. For example, in wild type paramyxoviruses, a typical RNA genome includes a 3'-leader region, six ORFs encoding the N, P, M, F, HN, and L proteins in the antisense direction and in this order, and a 5'-trailer region on the other end. The viral gene orientation in the genomic RNAs of the present invention is not restricted, but similarly to the wild type viruses, it is preferable that ORFs encoding the N, P, M, F, HN, and L proteins are arranged in this order, after the 3'-leader region, and before the 5'-trailer region. Certain types of viruses have different viral genes, but even in such cases, it is preferable that each gene be arranged as in the wild type, as described above. In general, viruses maintaining the N, P, and L genes can autonomously express genes from the RNA genome in cells, replicating the genomic RNA. Furthermore, by the action of genes such as the F and HN genes, which encode envelope proteins, and the M gene, infectious virions are formed and released to the outside of cells. Thus, such viruses become viruses with propagation ability. "With propagation ability" indicates that when a virus infects a host cell, the virus is amplified in the cell and infectious virions are produced. In the present invention, a foreign gene may be inserted into a protein-noncoding region in this genome, as required.

Further, a minus-strand RNA virus of this invention may be deficient in any of the wild type virus genes. For example, a virus that excludes the M, F, or HN gene, or any combination thereof, can be preferably used in this invention. Such viruses can be reconstituted, for example, by externally supplying the products of the deficient genes. Similar to wild type viruses, the viruses thus prepared adhere to host cells and cause cell fusion, but they cannot form daughter virions that retain the same infectivity as the original virus, because the virus genome introduced into cells is deficient in viral genes. Therefore, such viruses are useful as safe viruses that can only introduce genes once (for example, foreign genes). Examples of genes in which the genome may be deficient are the F gene and/or HN gene. For example, viruses can be reconstituted by transfecting host cells with a plasmid expressing a recombinant minus-strand RNA viral genome deficient in the F gene, along with an F protein expression vector and expression vectors for the NP, P, and L proteins (WO00/70055 and WO00/70070; Li, H.-O. et al., J. Virol. 74(14) 6564-6569 (2000)). Viruses can also be produced, for example, using host cells that have incorporated the F gene into their chromosomes. In these proteins, the amino acid sequences do not need to be the same as the viral sequences, and a mutant or homologous gene from another virus may be used as a substitute, so long as the activity in nucleic acid introduction is the same as, or greater than, that of the natural type.

Further, viruses that include an envelope protein other than that of the virus from which the viral genome was derived, may be prepared as viruses used in this invention. For example, when reconstituting a virus, a virus including a desired envelope protein can be generated by expressing an envelope protein other than the envelope protein encoded by the basic viral genome. Such proteins are not particularly limited. A desired protein that confers an ability to infect cells may be used. Examples of such proteins include the envelope proteins of other viruses, for example, the G protein of vesicular stomatitis virus (VSV-G). The VSV-G protein may be derived from an arbitrary VSV strain. For example, VSV-G proteins derived from Indiana serotype strains (J. Virology 39: 519-528 (1981)) may be used, but the present invention is not limited thereto. Furthermore, the virus of the present invention may include any arbitrary combination of envelope proteins derived from other viruses. Preferred examples of such proteins are envelope proteins derived from viruses that infect human cells. Such proteins are not particularly limited, and include retroviral amphotropic envelope proteins and the like. For example, the envelope proteins derived from mouse leukemia virus (MuLV) 4070A strain can be used as the retroviral amphotropic envelope proteins. In addition, envelope proteins derived from MuMLV 10A1 strain may also be used (for example, pCL-10A1 (Imgenex) (Naviaux, R. K. et al., J. Virol. 70:5701-5705 (1996)). The proteins of *Herpesviridae* include, for example, gB, gD, gH, and gp85 proteins of herpes simplex viruses, and gp350 and gp220 proteins of EB virus. The proteins of *Hepadnaviridae* include the S protein of hepatitis B virus. These proteins may be used as fusion proteins in which the extracellular domain is linked to the intracellular domain of the F or HN protein. As described above, the viruses used in this invention include pseudotype viruses that include envelope proteins, such as VSV-G, derived from viruses other than the virus from which the genome was derived. If the viruses are designed such that these envelope proteins are not encoded in RNA genomes, the proteins will never be expressed after virion infection of the cells.

Furthermore, the viruses used in this invention may be, for example, viruses that include on the envelope surface thereof, proteins such as adhesion factors capable of adhering to specific cells, ligands, receptors, antibodies or fragments, or virusesthat include a chimeric protein with these proteins in the extracellular domain and polypeptides derived from the virus envelope in the intracellular domain. These proteins may be encoded in the viral genome, or supplied through the expression of genes not in the viral genome (for example, genes carried by other expression vectors, or genes in the host chromosomes) at the time of viral reconstitution.

Further, in the viruses, any viral gene contained in the virus may be modified from the wild type gene in order to reduce the immunogenicity caused by viral proteins, or to enhance RNA transcriptional or replicational efficiency, for example. Specifically, for example, modifying at least one of the replication factors N, P, and L genes, is considered to enhance transcriptional or replicational function. Furthermore, although the HN protein, which is an envelope protein, has both hemagglutinin activity and neuraminidase activity, it is possible, for example, to improve viral stability in blood if the former activity is attenuated, and infectivity can be controlled if the latter activity is modified. Further, it is also possible to control membrane fusion ability by modifying the F protein. For example, the epitopes of the F protein and/or HN protein, which can be cell surface antigenic molecules, can be analyzed, and using this, viruses with reduced antigenicity to these proteins can be prepared.

Furthermore, the minus-strand RNA virus may be deficient in one or more accessory genes. For example, by knocking out the V gene, one of the SeV accessory genes, the pathogenicity of SeV toward hosts such as mice is remarkably reduced, without hindering gene expression and replication in cultured cells (Kato, A. et al., 1997, J. Virol. 71; 7266-7272; Kato, A. et al., 1997, EMBO J. 16: 578-587; Curran, J. et al., WO01/04272, EP1067179).

Minus-strand RNA viruses can be made, for example, to play a role as a vector to introduce foreign genes that are expected to have a synergistic effect with their own anticancer effect, These vectors do not have a DNA phase and carry out transcription and replication only in the host cytoplasm, and consequently, chromosomal integration does not occur (Lamb, R.A. and Kolakofsky, D., Paramyxoviridae: The viruses and their replication. In: Fields BN, Knipe DM, Howley PM, (eds). Fields of Virology. Vol. 2. Lippincott - Raven Publishers: Philadelphia, 1996, pp. 1177-1204). Therefore, safety issues such as transformation and immortalization due to chromosomal abberation do not occur. This characteristic of minus-strand RNA viruses contributes greatly to safety when it is used as a vector, For example, results on foreign gene expression show that even after multiple continuous passages of SeV, almost no nucletide mutation is observed. This suggests that the viral genome is highly stable and the inserted foreign genes are stably expressed over long periods of time (Yu, D. et al., Genes Cells 2, 457-466 (1997)). Further, there are qualitative advantages associated with SeV not having a capsid structural protein, such as packaging flexibility and insert gene size. Thus, minus-strand RNA viruses can be made to play an additional role as a highly efficient vector for human gene therapy. For example, SeV with propagation ability are capable of introducing foreign genes of up to at least 4 kb in size, and can simultaneously express two or more kinds of genes by adding the transcriptional units.

Further, SeV is known to be pathogenic in rodents causing pneumonia, but is not pathogenic for human, This is also supported by a previous report that nasal administration of wild type SeV does not have severely harmful effects on non-human primates (Hurwitz, J.L. et al., Vaccine 15: 533-540, 1997). These SeV characteristics suggest that SeV can be applied therapeutically on humans, supporting the proposition that SeV are a promising choice of gene therapy for cancer.

Although not required, minus-strand RNA viruses of this invention may encode foreign genes in their genomic RNA. A recombinant virus harboring a foreign gene is obtained by inserting a foreign gene into an above-described viral genome. The foreign gene can be any desired gene expected to have a synergistic effect and such with the anticancer effect of the RNA virus of this invention, and may be a gene that encodes a naturally-occurring protein, or protein modified from a naturally-occurring protein by deletion, substitution, or insertion of amino acid residues. The foreign gene can be inserted at any desired position in a protein-noncoding region of the virus genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region. Further, in genomes deficient in the F or HN gene or the like, nucleic acids encoding the foreign genes can be inserted into those deficient regions. When introducing a foreign gene into a paramyxovirus, it is desirable to insert the gene such that the chain length of the polynucleotide to be inserted into the genome will be a multiple of six (Journal of Virology, Vol. 67, No.8, 4822-4830, 1993). An E-I-S sequence should be arranged between the inserted foreign gene and the viral ORF. Two or more genes can be inserted in tandem via E-I-S sequences.

Expression levels of a foreign gene carried in a minus-strand RNA virus can be controlled using the type of transcriptional initiation sequence added upstream (to the 3'-side of the negative strand) of the gene (WO01/18223). The expression levels can also be controlled by the position at which the foreign gene is inserted in the genome: the nearer to the 3'-end of the negative strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high foreign gene expression level is thought to be advantageous, it is preferable to link the foreign gene to a highly efficient transcriptional initiation sequence, and to insert it near the 3'-end of the negative strand genome. Specifically, a foreign gene is inserted between the 3'-leader region and the viral protein ORF closest to the 3'-end. Alternatively, a foreign gene may be inserted between the ORFs of the viral gene closest to the 3'-end and the second closest viral gene. In wild type paramyxoviruses, the viral protein gene closest to the 3'-end of the genome is the N gene, and the second closest gene is the P gene. Alternatively, when a high level of expression of the introduced gene is undesirable, the gene expression level from the virus can be suppressed to obtain an appropriate effect, for example, by inserting the foreign gene at a site in the virus as close as possible to the 5'-side of the negative strand, or by selecting an inefficient transcriptional initiation sequence.

To prepare a minus-strand RNA virus, a cDNA encoding a genomic RNA of a virus is transcribed in mammalian cells, in the presence of viral proteins (i.e., N, P, and L proteins) essential for reconstitution of an RNP, which is a component of a virus. Viral RNP can be reconstituted by producing either the negative strand genome (that is, the same antisense strand as the viral genome) or the positive strand (the sense strand encoding the viral proteins). Production of the positive strand is preferable for increased efficiency of minus-strand RNA virus reconstitution. The RNA terminals preferably reflect the terminals of the 3'-leader sequence and 5'-trailer sequence as accurately as possible, as in the natural viral genome. To accurately regulate the 5'-end of the transcript, for example, the RNA polymerase may be expressed within a cell using the recognition sequence of T7 RNA polymerase as a transcription initiation site. To regulate the 3'-end of the transcript, for example, a self-cleaving ribozyme can be encoded at the 3'-end of the transcript, allowing accurate cleavage of the 3'-end with this ribozyme (Hasan, M. K. et al., J. Gen. Virol. 78: 2813-2820, 1997; Kato, A. et al., 1997, EMBO J. 16: 578-587; and Yu, D. et al., 1997, Genes Cells 2: 457-466).

For example, a recombinant Sendai virus carrying a foreign gene can be constructed as follows, according to descriptions in: Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; or the like.

First, a DNA sample including a cDNA sequence of an objective foreign gene is prepared. The DNA sample is preferably one that can be confirmed to be a single plasmid by electrophoresis at a concentration of 25 ng/μl or more. The following explains the case of using a *Not*I site to insert a foreign gene into a DNA encoding a viral genomic RNA, with reference to examples. When a *Not*I recognition site is included in a target cDNA nucleotide sequence, the nucleotide sequence is altered using site-directed mutagenesis or the like, such that the encoded amino acid sequence does not change, and the *Not*I site is preferably excised in advance. The objective gene fragment is amplified from this sample by PCR, and then recovered. By adding the *Not*I site to the 5' regions of a pair of primers, both ends of the amplified fragments become *Not*I sites. E-I-S sequences are designed to be included in primers such that, after a foreign gene is inserted into the viral genome, one E-I-S sequence each is placed between the ORF of the foreign gene, and either side of the ORFs of the viral genes.

For example, to guarantee cleavage with *Not*I, the forward side synthetic DNA sequence has a form in which any desired sequence of not less than two nucleotides (preferably four nucleotides not including a sequence derived from the *Not*I recognition site, such as GCG and GCC, and more preferably ACTT) is selected at the 5'-side, and a *Not*I recognition site 'gcggcegc' is added to its 3'-side. To that 3'-side, nine arbitrary nucleotides, or nine plus a multiple of six nucleotides are further added as a spacer sequence. To the further 3' of this, a sequence corresponding to about 25 nucleotides of the ORF of a desired cDNA, including and counted from the initiation codon ATG, is added. The 3'-end of the forward side synthetic oligo DNA is preferably about 25 nucleotides, selected from the desired cDNA such that the final nucleotide becomes a G or C.

For the reverse side synthetic DNA sequence, no less than two arbitrary nucleotides (preferably four nucleotides not including a sequence derived from a *Not*I recognition site, such as GCG and GCC, and more preferably ACTT) are selected from the 5'-side, a *Not*I recognition site 'gcggccgc' is added to its 3'-side, and to that 3' is further added an oligo DNA insert fragment for adjusting the length. The length of this oligo DNA is designed such that the chain length of the *Not*I fragment of the final PCR-amplified product will become a multiple of six nucleotides (the so-called "rule of six"); Kolakofski, D., et al., J. Virol. 72:891-899, 1998; Calain, P. and Roux, L., J. Virol. 67:4822-4830, 1993; Calain, P. and Roux, L., J. Virol. 67: 4822-4830, 1993). When adding an E-I-S sequence to this primer, to the 3'-side of the oligo DNA insertion fragment is added the complementary strand sequence of the Sendai virus S, I, and E sequences, preferably 5'-CTTTCACCCT-3' (SEQ ID NO: 1), 5'-AAG-3', and 5'-TTTTTCTTACTACGG-3' (SEQ ID NO: 2), respectively; and further to this 3'-side is added a complementary strand sequence corresponding to about 25 nucleotides, counted backwards from the termination codon of a desired cDNA sequence, whose length has been selected such that the final nucleotide of the chain becomes a G or C, to make the 3'-end of the reverse side synthetic DNA.

PCR can be performed according to conventional methods, using Taq polymerase or other DNA polymerases. Objective amplified fragments may be digested with *Not*I, and then inserted into the *Not*I site of plasmid vectors such as pBluescript. The nucleotide sequences of PCR products thus obtained are confirmed with a sequencer, and plasmids that include the correct sequence are selected. The inserted fragment is excised from these plasmids using *Not*I, and cloned into the *Not*I site of a plasmid composed of genomic cDNA. A recombinant Sendai virus cDNA can also be obtained by inserting the fragment directly into the *Not*I site of a genomic cDNA, without using a plasmid vector.

For example, a recombinant Sendai virus genomic cDNA can be constructed according to methods described in the literature (Yu, D. et al., Genes Cells 2: 457-466, 1997; Hasan, M. K. et al., J. Gen. Virol. 78: 2813-2820, 1997). For example, an 18 bp spacer sequence (5'-(G)-CGGCCGCAGATCTTCACG-3') (SEQ ID NO: 3), including a *Not*I restriction site, is inserted between the leader sequence and the ORF of N protein of the cloned Sendai virus genomic cDNA (pSeV(+)), obtaining plasmid pSeV18⁺b(+), which includes an auto-cleavage ribozyme site derived from the antigenomic strand of delta hepatitis virus (Hasan, M. K. et al., 1997, J. General Virology 78: 2813-2820). A recombinant Sendai virus cDNA including a desired foreign gene can be obtained by inserting a foreign gene fragment into the *Not*I site of pSeV18⁺b(+).

A virus can be reconstituted by transcribing a DNA encoding a genomic RNA of a recombinant virus thus prepared, in cells in the presence of the above-described viral proteins (L, P, and N).

Moreover, the recombinant viruses can also be reconstituted by methods known in the art (WO97/16539; WO97/16538; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). With these methods, minus-strand RNA viruses including parainfluenza virus, vesicular stomatitis virus, rabies virus, measles virus, rinderpest virus, and Sendai virus can be reconstituted from DNA. The viruses of this invention can be reconstituted according to these methods. When a viral DNA is made F gene-, HN gene-, and/or M gene-deficient, such DNAs do not form infectious virions as is. However, infectious virions can be formed by separately introducing host cells with these deficient genes, and/or genes encoding the envelope proteins of other viruses, and then expressing these genes therein.

Specifically, the viruses can be prepared by the steps of: (a) transcribing cDNAs encoding genomic RNAs of minus-strand RNA viruses (negative strand RNAs), or complementary strands thereof (positive strands), in cells expressing N, P, and L proteins; and (b) harvesting culture supernatants thereof including the produced minus-strand RNA viruses. For transcription, a DNA encoding a genomic RNA is linked downstream of an appropriate promoter. The genomic RNA thus transcribed is replicated in the presence of N, L, and P proteins to form an RNP complex. Then, in the presence of M, HN, and F proteins, virions enclosed in an envelope are formed. For example, a DNA encoding a genomic RNA can be linked downstream of a T7 promoter, and transcribed to RNA by T7 RNA polymerase. Any desired promoter can be used as a promoter, in addition to those including a T7 polymerase recognition sequence. Alternatively, RNA transcribed *in vitro* may be transfected into cells.

Enzymes essential for the initial transcription of genomic RNA from DNA, such as T7 RNA polymerase, can be supplied by introducing the plasmid or viral vectors that express them, or, for example, by incorporating the RNA polymerase gene into a chromosome of the cell so as to enable induction of its expression, and then inducing expression at the time of viral reconstitution. Further, genomic RNA and viral proteins essential for virus reconstitution are supplied, for example, by introducing the plasmids that express them. In supplying these viral proteins, helper viruses such as the wild type or certain types of mutant minus-strand RNA viruses can be used.

Methods for introducing DNAs expressing the genomic RNAs into cells include, for example, (i) methods for making DNA precipitates which target cells can internalize; (ii) methods for making complexes including DNAs that are suitable for internalization by target cells, and have a low-cytotoxic positive charge; and (iii) methods for using electric pulses to instantaneously create holes in the target cell membrane, which are of sufficient size for DNA molecules to pass through.

In the context of method (ii), various transfection reagents can be used. For example, DOTMA (Roche), Superfect (QIAGEN #301305), DOTAP, DOPE, DOSPER (Roche #1811169), and the like can be cited. Regarding method (i), for example, transfection methods using calcium phosphate can be cited, and although DNAs transferred into cells by this method are internalized by phagosomes, a sufficient amount of DNA is known to enter the nucleus (Graham, F. L. and Van Der Eb, J., 1973, Virology 52: 456; Wigler, M. and Silverstein, S., 1977, Cell 11: 223). Chen and Okayama investigated the optimization of transfer techniques, reporting that (1) incubation conditions for cells and coprecipitates are 2 to 4% $CO_2$ 35°C, and 15 to 24 hours, (2) the activity of circular DNA is higher than linear DNA, and (3) optimal precipitation is obtained when the DNA concentration in the precipitate mixture is 20 to 30 $\mu$g/ml (Chen, C. and Okayama, H., 1987, Mol. Cell. Biol. 7: 2745). The methods af (ii) are suitable for transient transfections. Methods for performing transfection by preparing a DEAE-dextran (Sigma #D-9885 M.W. 5x $10^5$) mixture with a desired DNA concentration ratio have been known for a while. Since most complexes are decomposed in endosomes, chloroquine may also be added to enhance the effect (Calos, M. P., 1983, Proc. Natl. Acad. Sci. USA 80: 3015). The methods of (iii) are referred to as electroporation methods, and are used more in general than methods (i) or (ii) because they are not cell-selective. The efficiency of these methods is presumed to be good under optimal conditions for: the duration of pulse electric current, shape of the pulse, potency of electric field (gap between electrodes, voltage), conductivity of buffer, DNA concentration, and cell density.

Of the above three categories, the methods of (ii) are simple to operate and facilitate examination of many samples using a large amount of cells, making transfection reagents suitable for the transduction into cells of DNA for virus reconstitution. Preferably, the Superfect Transfection Reagent (QIAGEN, Cat No. 301305), or the DOSPER Liposomal Transfection Reagent (Roche, Cat No. 1811169) is used; however, the transfection reagents are not limited to these.

Specifically, virus reconstitution from cDNA can be carried out, for example, as follows:

In a plastic plate of about 6 to 24 wells, or a 100-mm Petri dish or the like, simian kidney-derived LLC-MK2 cells (ATCC CCL-7) are cultured up to about 100% confluency, using minimum essential medium (MEM) including 10% fetal calf serum (FCS) and antibiotics (100 units/ml penicillin G and 100 $\mu$g/ml streptomycin). Then they are infected with, for example, two plaque forming units (PFU)/cell of the recombinant vaccinia virus vTF7-3, which expresses T7 RNA polymerase and has been inactivated by 20-minutes of UV irradiation in the presence of 1 $\mu$g/ml psoralen (Fuerst,

T. R. et al., Proc. Natl. Acad. Sci. USA 83: 8122-8126,1986; Kato, A. et al., Genes Cells 1: 569-579, 1996). The amount of psoralen added and the UV irradiation time can be appropriately adjusted. One hour after infection, 2 to 60 μg, and more preferably 3 to 20 μg, of DNA encoding the genomic RNA of a recombinant Sendai virus is transfected along with the plasmids expressing trans-acting viral proteins essential for viral RNP production (0.5 to 24 μg of pGEM-N, 0.25 to 12 μg of pGEM-P, and 0.5 to 24 μg of pGEM-L) (Kato, A. et al., Genes Cells 1: 569-579, 1996), using the lipofection method or the like with Superfect (QIAGEN). For example, the ratio of the amounts of expression vectors encoding the N, P, and L proteins is preferably 2:1:2, and the plasmid amounts are appropriately adjusted in the range of 1 to 4 μg of pGEM-N, 0.5 to 2 μg of pGEM-P, and 1 to 4 μg ofpGEM-L.

The transfected cells are cultured, as desired, in serum-free MEM composed of 100 μg/ml of rifampicin (Sigma) and cytosine arabinoside (AraC), more preferably only 40 μg/ml of cytosine arabinoside (AraC) (Sigma). Optimal drug concentrations are set so as to minimize cytotoxicity due to the vaccinia virus, and to maximize virus recovery rate (Kato, A. et al., 1996, Genes Cells 1: 569-579). After culturing for about 48 to 72 hours after transfection, cells are harvested, and then disintegrated by repeating freeze-thawing three times. LLC-MK2 cells are re-transfected with the disintegrated materials including RNP, and cultured. Alternatively, the culture supernatant is recovered, added to a culture solution of LLC-MK2 cells to infect them, and the cells are then cultured. Transfection can be conducted by, for example, forming a complex with lipofectamine, polycationic liposome, or the like, and introducing the complex into cells. Specifically, various transfection reagents can be used. For example, DOTMA (Roche), Superfect (QIAGEN #301305), DOTAP, DOPE, and DOSPER (Roche #1811169) may be cited. In order to prevent decomposition in the endosome, chloroquine may also be added (Calos, M. P., 1983, Proc. Natl. Acad. Sci. USA 80: 3015). In cells transduced with RNP, viral gene expression from RNP and RNP replication progress, and the virus is amplified. By diluting the viral solution thus obtained (for example, $10^6$-fold), and then repeating the amplification, the vaccinia virus vTF7-3 can be completely eliminated. Amplification is repeated, for example, three or more times. Viruses thus obtained can be stored at -80°C. In order to reconstitute a virus having no propagation ability and lacking a gene encoding an envelope protein, LLC-MK2 cells expressing the envelope protein may be used for transfection, or a plasmid expressing the envelope protein may be cotransfected. Alternatively, a defective type virus can be amplified by culturing the transfected cells overlaid with LLK-MK2 cells expressing the envelope protein (see WO00/70055 and WO00/70070).

Titers of viruses thus recovered can be determined, for example, by measuring CIU (Cell-Infected Unit) or hemagglutination activity (HA) (WO00/70070; Kato, A. et al., 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of viruses carrying GFP (green fluorescent protein) marker genes and the like can be quantified by directly counting infected cells, using the marker as an indicator (for example, as GFP-CIU). Titers thus measured can be treated in the same way as CIU (WO00/70070).

So long as a virus can be reconstituted, the host cells used in the reconstitution are not particularly limited. For example, in the reconstitution of Sendai viruses and the like, cultured cells such as LLC-MK2 cells and CV-1 cells derived from monkey kidney, BHK cells derived from hamster kidney, and cells derived from humans can be used. By expressing suitable envelope proteins in these cells, infectious virions including the proteins in the envelope can also be obtained. Further, to obtain a large quantity of a Sendai virus, a virus obtained from an above-described host can be infected to embrionated hen eggs, to propagate the virus. Methods for manufacturing viruses using hen eggs have already been developed (Nakanishi, et al., ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for nine to twelve days at 37 to 38°C to grow an embryo. After the virus is inoculated into the allantoic cavity, the egg is cultured for several days (for example, three days) to proliferate the virus. Conditions such as the period of culture may vary depending upon the recombinant Sendai virus being used. Then, allantoic fluids including the virus are recovered. Separation and purification of a Sendai virus from allantoic fluids can be performed according to a usual method (Tashiro, M., "Virus Experiment Protocol," Nagai, Ishihama, ed., Medical View Co., Ltd., pp. 68-73, (1995)).

For example, the construction and preparation of Sendai viruses defective in F gene can be performed as described below (see WO00/70055 and WO00/70070).

<1> Construction of a genomic cDNA of an F-gene defective Sendai virus, and a plasmid expressing F gene:

A full-length genomic cDNA of Sendai virus (SeV), the cDNA of pSeV18⁺ b (+) (Hasan, M. K. et al., 1997, J. General Virology 78: 2813-2820) ("pSeV18⁺ b (+)" is also referred to as "pSeV18⁺"), is digested with SphI/KpnI to recover a fragment (14673 bp), which is cloned into pUC18 to prepare plasmid pUC18/KS. Construction of an F gene-defective site is performed on this pUC18/KS. An F gene deficiency is created by a combination of PCR-ligation methods, and, as a result, the F gene ORF (ATG-TGA = 1698 bp) is removed. Then, for example, 'atgcatgccggcagatga (SEQ ID NO: 4)' is ligated to construct an F gene-defective type SeV genomic cDNA (pSeV18⁺/ΔF). A PCR product formed in PCR by using the pair of primers [forward: 5'-gttgagtactgcaagagc/SEQ ID NO: 5, reverse:

5'-tttgccggcatgcatgtttcccaaggggagagttttgcaacc/SEQ ID NO: 6] is connected upstream of F, and a PCR product formed using the pair of primers [forward: 5'-atgcatgccggcagatga/SEQ ID NO: 7, reverse: 5'-tgggtgaatgagagaatcagc/SEQ ID NO: 8] is connected downstream of the F gene with *Eco*T22I. The plasmid thus obtained is digested with *Sac*I and *Sal*I to recover a 4931 bp fragment of the region including the F gene-defective site, which is cloned into pUC18 to form pUC18/dFSS, This pUC 18/dFSS is digested with *Dra*III, the fragment is recovered, replaced with the *Dra*III fragment of the region including the F gene of pSeV18+, and ligated to obtain the plasmid pSeV18+/ΔF.

A foreign gene is inserted, for example, into the *Nsi*I and *Ngo*MIV restriction enzyme sites in the F gene-defective site of pUC18/dFSS. For this, a foreign gene fragment may be, for example, amplified using an NsiI-tailed primer and an NgoMIV-tailed primer.

<2> Preparation of helper cells that induce SeV-F protein expression:

To construct an expression plasmid of the Cre/loxP induction type that expresses the Sendai virus F gene (SeV-F), the SeV-F gene is amplified by PCR, and inserted to the unique *Swa*I site of the plasmid pCALNdlw (Arai, T. et al., J. Virology 72, 1998, p1115-1121), which is designed to enable the inducible expression of a gene product by Cre DNA recombinase, thus constructing the plasmid pCALNdLw/F.
To recover infectious virions from the F gene-defective genome, a helper cell line expressing SeV-F protein is established. The monkey kidney-derived LLC-MK2 cell line, which is commonly used for SeV propagation, can be used as the cells, for example. LLC-MK2 cells are cultured in MEM supplemented with 10% heat-inactivated fetal bovine serum (FBS), penicillin G sodium (50 units/ml), and streptomycin (50 $\mu$g/ml) at 37°C in 5% $CO_2$. Since the SeV-F gene product is cytotoxic, the above-described plasmid pCALNdLw/F, which was designed to enable inducible expression of the F gene product with Cre DNA recombinase, is transfected to LLC-MK2 cells by the calcium phosphate method (using a mammalian transfection kit (Stratagene)), according to protocols well known in the art.
The plasmid pCALNdLw/F (10 $\mu$g) is introduced into LLC-MK2 cells grown to 40% confluency using a 10-cm plate, and the cells are then cultured in MEM (10 ml) including 10% FBS, in a 5% $CO_2$ incubator at 37°C for 24 hours. After 24 hours, the cells are detached and suspended in the medium (10 ml). The suspension is then seeded into five 10-cm dishes, 5 ml into one dish, 2 ml each into two dishes, and 0.2 ml each into two dishes, and cultured in MEM (10 ml) including G418 (GIBCO-BRL) (1200 $\mu$g/ml) and 10% FBS. The cells were cultured for 14 days, exchanging the medium every two days, to select cell lines into which the gene is stably introduced. The cells grown from the above medium that show G418 resistance are recovered using a cloning ring. Culture of each clone thus recovered is continued in 10-cm plates until confluent.
After the cells have grown to confluency in a 6-cm dish, F protein expression can be induced by infecting the cells with adenovirus AxCANCre, for example, at MOI = 3, according to the method of Saito, *et al.* (Saito et al., Nucl. Acids Res. 23: 3816-3821 (1995); Arai, T. et al., J. Virol 72, 1115-1121 (1998)).

<3> Reconstruction and amplification of F gene-deficient SeV virus:

The above-described plasmid pSeV18+/ΔF inserted with the foreign gene is transfected into LLC-MK2 cells by the procedure described below. LLC-MK2 cells are seeded on 100-mm dishes at 5 x $10^6$ cells/dish. To transcribe the genomic RNA using T7 RNA polymerase, the cells are cultured for 24 hours, and then recombinant vaccinia virus, which expresses T7 RNA polymerase (PLWUV-VacT7: Fuerst, T.R. et al., Proc. Natl. Acad. Sci. USA 83, 8122-8126 (1986)) and is treated with psoralen and long-wavelength ultraviolet light (365 nm) for 20 minutes, is inoculated to the cells at a MOI of about 2 at room temperature for one hour. The ultraviolet light irradiation to the vaccinia virus can be achieved, for example, by using UV Stratalinker 2400 with five 15-watt bulbs (Catalog No. 400676 (100V); Stratagene, La Jolla, CA, USA). After the cells are washed with serum-free MEM, plasmid expressing the genomic RNA and expression plasmids each expressing N, P, L, F, or HN protein of the minus-strand RNA virus are transfected into the cells using an appropriate lipofection reagent. The plasmid ratio is preferably, but is not limited to, 6:2:1:2:2:2 in this order. For example, the expression plasmid for the genomic RNA, and the expression plasmids each of which expresses N, P, or L protein, or F and HN proteins (pGEM/NP, pGEM/P, pGEM/L, and pGEM/F-HN; WO00/70070, Kato, A. et al., Genes Cells 1, 569-579 (1996)) are transfected at amounts of 12, 4, 2, 4, and 4 $\mu$g/dish, respectively. After a few hours of culture, the cells are washed twice with serum-free MEM, and then cultured in MEM supplemented with 40 $\mu$g/ml cytosine β-D-arabinofuranoside (AraC: Sigma, St. Louis, MO) and 7.5 $\mu$g/ml trypsin (Gibco-BRL, Rockville, MD). The cells are recovered, and the resulting pellet is suspended in OptiMEM ($10^7$ cells/ml). The suspension is subjected to three freeze-thaw cycles, and mixed with lipofection reagent DOSPER (Boehringer Mannheim) ($10^6$ cells/25 $\mu$l DOSPER). After the mixture is allowed to stand at room temperature for 15 minutes, it is transfected to F-expressing helper cells ($10^6$ cells/well in 12-well-plate) cloned as described above. The cells are cultured in serum-free MEM (containing 40 $\mu$g/ml AraC and 7.5 $\mu$g/ml trypsin), and the supernatant is collected. Viruses deficient in genes other than F, for example, HN and M genes, can be prepared by a similar method as described above.

Instead of a recombinant virus, a naturally derived virus may also be used as a minus-strand RNA virus of the present invention, and one can refer to "Uirusu Jikken-gaku kakuron (Virology Experiments in detail)", second revised edition (edited by Researcher's Associates at the National Institute of Health, Maruzen, 1982) for methods of purifying, multiplying, and obtaining isolated strains of each of the RNA viruses. For example, each type of parainfluenza virus, such as Sendai virus belonging to the *Paramyxoviridae* family, can propagate well in primary culture cells of simian kidney (MK2), human embryonic lung, kidney, and amnion, and in trypsin-supplemented Vero cells (same as above, p334; Itoh H. et al., Jap. J. Med. Sci. Biol. 23, 227 (1970)), and can then be collected. Purification can be carried out by the sucrose density gradient centrifugation method, and equilibrium centrifugation method (p336). Measles virus can propagate well in various cells derived from monkeys (Matsumoto M., Bact. Rev. 30, 152 (1966)), and while Vero cells are most widely used, it can be propagated using CV1, FL, KB, HeLa, HEp2, and such (p351). Viruses belonging to the *Rhabdoviridae* family, such as the rabies virus, are propagated by tissue culture methods using BHK, CE, Vero cells, and the like. Purification methods involve the steps of adjusting the pH of a culture solution on the third to fourth day of infection to 7.4 or more, and concentrating the solution after removing cell debris by low-speed centrifugation (p376). Viruses belonging to the *Arenaviridae* family, such as the lassa virus, propagate well in most cultured cells that are subcultured *in vitro,* and it can be propagated by infection into HK-21/13S cells, followed by culturing as a suspension in agar (Sedwik W. D., J. Virol. 1, 1224 (1967)) (p240). Viruses belonging to the *Orthomyxoviridae* family, such as the influenza virus, can be propagated in developing hen eggs and MDCK cells (p295), Purification can be carried out by methods such as centrifugation, and adsorption to and release from red blood cells (Laver W. G.., Fundamental Techniques in Virology, 82 (1969)) (p317).

There is no limitation on the foreign gene to be introduced using the minus-strand RNA virus, and examples of naturally occurring proteins include, for example, hormones, cytokines, growth factors, receptors, intracellular signaling molecules, enzymes, and peptides. The proteins may be secretory proteins, membrane proteins, cytoplasmic proteins, nuclear proteins, and the like. Artificial proteins include, for example, fusion proteins of chimeric toxins and such, dominant negative proteins (including soluble receptor molecules or membrane-bound dominant negative receptors), cell surface molecules and truncated cell adhesion molecules. The proteins may also be proteins to which a secretory signal, membrane-localization signal, nuclear translocation signal, or the like has been attached. Functions of a particular gene can be suppressed by expressing antisense RNA molecules, RNA-cleaving ribozymes, or the like as the introduced gene. When a virus of this invention is prepared using a gene for treating diseases as the foreign gene, gene therapy can be performed by administering this virus.

According to the method for producing viruses as described herein, the virus of the present invention can be released into extracellular fluid of virus producing cells at a titer of, for example, $1 \times 10^5$ CIU/ml or higher, preferably $1 \times 10^6$ CIU/ml or higher, more preferably $5 \times 10^6$ CIU/ml or higher, more preferably $1 \times 10^7$ CIU/ml or higher, more preferably $5 \times 10^7$ CIU/ml or higher, more preferably $1 \times 10^8$ CIU/ml or higher, and more preferably $5 \times 10^8$ CIU/ml or higher. The titer of virus can be determined according to methods described herein or elsewhere (Kiyotani, K. et al., Virology 177(1), 65-74 (1990); and WO00/70070).

The recovered viruses can be purified to be substantial pure. The purification can be achieved using known purification/separation methods, including filtration, centrifugation, adsorption, and column purification, or any combinations thereof. The phrase "substantially pure" means that the virus component constitutes a major proportion of a solution comprising the virus. For example, a viral composition can be confirmed to be substantially pure by the fact that the proportion of protein contained as the viral component to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for, for example, the paramyxovirus include methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorbing to fucose sulfate-containing polysaccharide and/or degradation products thereof (WO97/32010), but are not limited thereto.

The minus-strand RNA viruses of the present invention may be, for example, infectious virions or non-infectious virions. The RNA viruses of the present invention may also be genomic RNA-protein complexes (ribonucleoprotein complexes; RNPs). When using such virions or complexes, these virions or complexes are preferably mixed with a lipofection reagent and administered *in vivo.* For example, virions and complexes can be mixed with lipofectamine or a polycationic liposome and this mixture can be administered *in vivo* (WO00/70055). In this method, various transfection reagents can be used. Examples include DOTMA (Boehringer), Superfect (QIAGEN #301305), DOTAP, DOPE, and DOSPER (Boehringer #1811169). Chloroquine can be added to prevent degradation in endosomes (Calos, M. P., 1983, Proc. Natl. Acad. Sci. USA 80: 3015).

In the production of an anticancer agent comprising a minus-strand RNA virus in the present invention, the minus-strand RNA virus can be mixed (combined) as necessary with a desired pharmaceutically acceptable carrier or medium.

Specifically, the present invention provides methods for producing an anticancer agent, which comprises the step of mixing a minus-strand RNA virus with a pharmaceutically acceptable carrier or medium.

The "pharmaceutically acceptable carrier or medium" refers to materials that can be administered together with a minus-strand RNA virus and which do not significantly inhibit infection by the minus-strand RNA virus. Such carrier or medium includes, for example, deionized water, sterilized water, sodium chloride solution, dextrose solution, culture medium, serum, phosphate buffered saline (PBS), and Ringer's solution containing dextrose, sodium chloride, and lactic acid, and they may be appropriately combined with a minus-strand RNA virus in formulations. Furthermore, they may be concentrated by centrifugation when necessary, and resuspended in a physiological solution such as a culture solution or physiological saline solution. They may also include membrane stabilizers for liposomes (for example, sterols such as cholesterol) or antioxidants (for example, tocopherol or vitamin E). In addition, vegetable oils, suspending agents, detergents, stabilizers, biocidal agents, and such may also be included. Preservatives and other additives can also be added. A composition of the present invention may take the form of an aqueous solution, capsule, suspension, syrup, or such. A virus composition of the present invention may be a composition in the form of a solution, freeze-dried product, or aerosol. In the case of a freeze-dried product, it may include sorbitol, sucrose, amino acids, various proteins, and such as stabilizers.

The minus-strand RNA viruses of the present invention have anticancer activity, and they can exhibit anticancer (carcinostatic) effects when administered to tumors (cancer tissues). Therefore, the present invention relates to a method for suppressing cancer (method for suppressing cancer cell growth, or method for treating cancer), which comprises the step of administering a minus-strand RNA virus to cancer tissues (*in vivo* administration).

For example, cancer therapy can be performed on cancer patients. This method comprises the step of administering a minus-strand RNA virus (an anticancer agent of the present invention). Specifically, the method comprises the step of administering a therapeutically effective amount of a minus-strand RNA virus to a patient. Use of the present method is expected to suppress the growth of cancer cells compared to when a minus-strand RNA virus of the present invention is not administered. The minus-strand RNA virus may not carry a foreign gene, or it may carry a gene (foreign gene) encoding one or more cancer antigens, immunostimulatory cytokines, proteins that inhibit angiogenesis, or such.

The present invention can be applied to desired solid cancers, and such examples include tongue cancer, gum cancer, malignant lymphoma, malignant melanoma, upper jaw cancer, nose cancer, nasal cavity cancer, larynx cancer, pharyngeal cancer, glioma, meningioma, glioma, lung cancer, breast cancer, pancreatic cancer, gastrointestinal cancer (esophageal cancer, stomach cancer, duodenal cancer, colon cancer), squamous cancer, adenocarcinoma, alveolar cell cancer, testis tumor, prostate cancer, thyroid cancer, liver cancer, ovarian cancer, rhabdomyosarcoma, fibrosarcoma, osteosarcoma, and chondrosarcoma. The target cancer is preferably epithelial cancer, and is more preferably skin cancer including skin squamous cancer, skin basal cell cancer, Bowen's disease, Paget's disease, and skin malignant melanoma.

The *in vivo* dose of the minus-strand RNA virus of this invention (anticancer agent of this invention) varies depending on the disease, patient's weight, age, sex, and symptom, purpose of administration, form of administered composition, administration method, introduced gene, and the like, but can be appropriately determined by those skilled in the art. The route of administration can be appropriately selected, and includes, for example, percutaneous, intranasal, transbronchial, intramuscular, intraperitoneal, intravenous, intraarticular, and subcutaneous administration. The administration may be local or systemic. It is preferred to administer the virus at a dose within the range of preferably about $10^5$ to about $10^{11}$ CIU/ml, more preferably about $10^7$ to about $10^9$ CIU/ml, and most preferably about $1 \times 10^8$ to about $5 \times 10^8$ CIU/ml, in a pharmaceutically acceptable carrier. The amount per dose for human is preferably $2 \times 10^5$ to $2 \times 10^{11}$ CIU, which is administered once or more within a range where the side effects are clinically acceptable. The same applies to the number of doses per day. Regarding nonhuman animals, for example, a dose converted from the above-described dose based on the body weight ratio between the subject animal and human or the volume ratio (e.g., mean value) of the target site for administration. In addition, when it becomes necessary to suppress the propagation of the transmissible minus-strand RNA virus after administration to subjects or cells due to the completion of treatment or the like, through the administration of an RNA-dependent RNA polymerase inhibitor, the propagation of the virus can be specifically suppressed without damaging the host.

The minus-strand RNA viruses of the present invention are preferably administered to the cancerous lesion of a patient. "Cancerous lesion" refers to a region of cancer tissue or its surrounding area (for example, within 5 mm of the cancer, or preferably within 3 mm of the cancer). The dose may be appropriately adjusted depending on the type and stage of the cancer, the presence or absence of an introduced gene, and such. Although antitumor effects are expected of minus-strand RNA viruses even if they do not carry foreign genes, higher synergistic effects can be expected by loading RNA viruses with, for example, an IFN-beta gene or soluble FGF receptor gene.

The minus-strand RNA virus can be administered one or more times as long as the side effects are clinically acceptable, and the same applies to the number of doses administered per day. Subjects receiving the administration are not particularly limited, and examples include birds and mammals (human and non-human mammals) such as chicken, quail, mouse, rat, dog, pig, cat, bovine, rabbit, sheep, goat, monkey, and human. When administering to non-human animals, for example, an amount calculated from the above-mentioned dose according to the body weight ratio between human and the animal of interest can be administered.

The present invention also relates to the use of a minus-strand RNA virus in cancer therapy. Furthermore, the present invention relates to the use of a minus-strand RNA virus in the production of anticancer agents (or carcinostatic agents, agents for suppressing cancer growth, or such).

In addition, the present invention relates to a package comprising a minus-strand RNA virus, which includes descriptions on the use of the minus-strand RNA virus for cancer suppression (as an anticancer agent). The minus-strand RNA virus may be suspended in a solution such as culture solution or physiological saline solution. "Use in cancer suppression" means that for example, a minus-strand RNA virus, or a composition comprising such RNA virus, is used in tumor growth suppression, cancer degeneration, cancer therapy, treatment and therapy of cancer patients, prolongation of the life of cancer patients, or as an anticancer agent. The descriptions can be printed directly on the package; alternatively, a piece of paper or a sticker containing the descriptions may be comprised in the package, The package may be a container comprising a minus-strand RNA virus, and in such case, the container may be, for example, a bottle, tube, plastic bag, vial, syringe, or such. Furthermore, the package of the present invention may comprise a bag, outer box, or such that stores the container. The package may comprise instructions describing the method for administering a minus-strand RNA virus, and may further comprise a syringe, catheter, and/or injection needle for administering the minus-strand RNA virus.

Furthermore, the present invention relates to a kit for treating cancer or for producing an anticancer agent, which comprises at least a minus-strand RNA virus as a component. In addition to a minus-strand RNA virus, a kit of the present invention may appropriately comprise, for example, carrier, physiological saline solution, buffer, bottle, tube, plastic bag, vial, and syringe. Instructions for use can also be packaged into the kit.

All references cited herein are incorporated as a part of this description.

Examples

Hereinbelow, the present invention will be specifically described with reference to the Examples, but it is not to be construed as being limited thereto.

[Example 1] Antitumor effects by a Sendai virus that does not carry a therapeutic gene

$1 \times 10^5$ cells of the melanoma cell line B16F 1 (ATCC CRL-6323) were inoculated subcutaneously into the ventral part of C57BL/6 mice (6 to 8-week old, female) (n=4), Five days (day 5) and 12 days (day 12) after inoculation, $1 \times 10^8$ PFU of Sendai virus that does not comprise any specific therapeutic gene (GFP-expressing SeV; SeV-GFP), Sendai virus expressing a human soluble FGF receptor (SeV-sFGFR), or Sendai virus expressing human soluble PDG-FR$\alpha$ (SeV-hsPDGFR$\alpha$) was injected into the tumor. Then, the tumor size was measured over time.

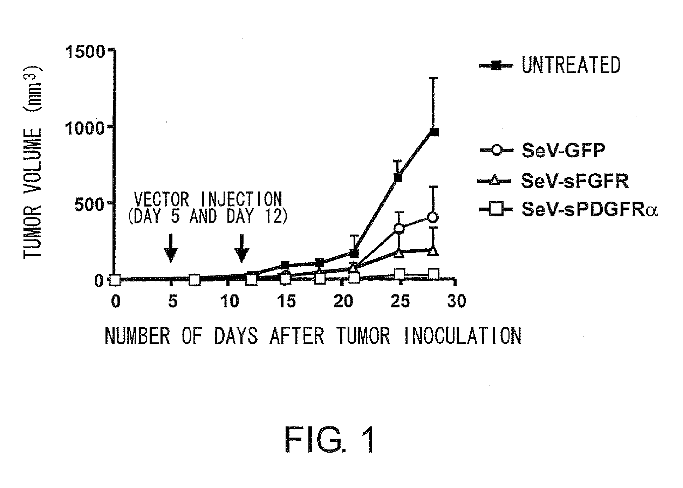

As a result, in all SeV-administered groups, the tumor size significantly decreased compared to the tumor size of the nonadministered group (Fig. 1). Therefore, *in vivo* administration of SeV was able to exhibit antitumor effects, even when the SeV did not carry any therapeutic gene. When SeV expressing a soluble FGF receptor was administered, a stronger effect of tumor growth suppression was confirmed as compared to that in the SeV-GFP-administered group. The antitumor effect was most significant when the soluble PDGFR$\alpha$-expressing SeV was administered, since the tumor size hardly increased.

The above revealed that SeV exhibits a sufficient antitumor effect even if it does not carry any foreign gene such as a therapeutic gene.

[Example 2] Antitumor effects by a therapeutic gene (IFN$\beta$)-comprising Sendai virus

This Example shows an example of a method for treating a tumor by *in vivo* and *ex vivo* administration of an RNA virus

A B16 melanoma-transplanted model that expresses MHC class I at only a very low level and exhibits poor immunogenicity was used as a tumor model. C57BL/6 mice (6- to 8-week-old; female) (CHARLES RIVER JAPAN, INC.) were used as the tumor model mice, and dendritic cells were collected from C57BL/6 mice (8-week-old; female) (CHARLES RIVER JAPAN, INC.). The dendritic cells were obtained by collecting bone marrow from thigh bones of C57BL/6 mice; removing T cells using SpinSep™, murine hematopoietic progenitor enrichment cocktail (anti-CD5 antibody, anti-CD45R antibody, anti-CD11b antibody, anti-Gr-1 antibody, anti-TER119 antibody, anti-7/4 antibody; Stem Cell technology); then culturing the cells for one week with the addition of IL-4 and GM-CSF. On day $0.1 \times 10^5/100$ $\mu$L of B16 melanoma cells were subcutaneously (s.c.) inoculated into the abdominal area of the mice. On days 10, 17, and 24, dendritic cells without stimulation for activation, dendritic cells activated with LPS (LPS DC), or dendritic cells activated by introducing SeV-GFP or SeV-IFN$\beta$ expressing mouse interferon $\beta$ (SeV GFP DC and SeV IFN$\beta$ DC, respectively)

were administered in the area surrounding the tumor. Simultaneously, another experiment was carried out, wherein the dendritic cells were administered after the pulsing with tumor antigens (tumor lysate obtained by freeze and thaw of B16). In addition, the antitumor effect of direct intratumoral injection of SeV-IFNβ into the tumor 10 days after tumor injection (day 10) was examined.

SeV was introduced into dendritic cells by infecting dendritic cells cultured for one week as described above with SeV-IFNβ at a MOI of 40, and culturing the cells for 8 hours. When pulsing dendritic cells with tumor antigens, dendritic cells cultured for one week as described above were recovered and pulsed with tumor lysate as the tumor antigens (DC: tumor lysate = 1:3), cultured for 18 hours, infected with SeV-IFNβ at a MOI of 40, and cultured for 8 hours. Then, these dendritic cells were recovered and administered at a cell number of 5 x $10^5$ to 10 x $10^5$ cells in an area surrounding the tumor of the mice.

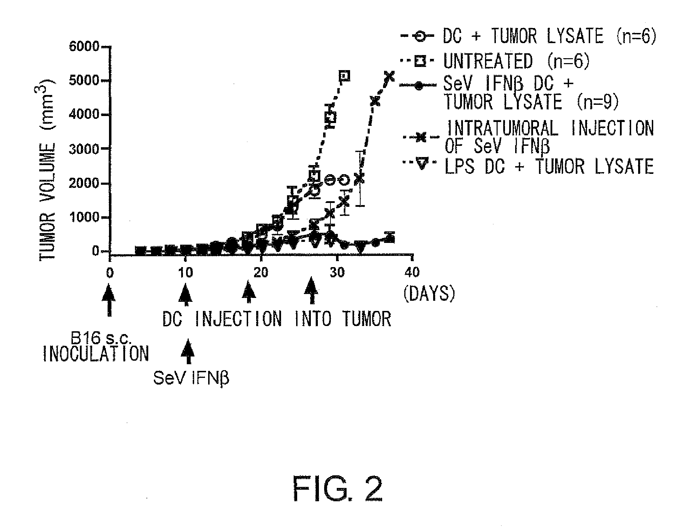

As shown in Fig. 2, SeV-IFNβ suppressed tumor growth, both in the case of direct intratumoral injection and in the case of *ex vivo* administration through dendritic cells In particular, a significantly strong tumor suppressing effect was observed in mice treated with DC/SeV-IFNβ.

The antitumor effect in each of the therapeutic groups described above was closely examined. To assay natural killer (NK) cell activity, spleens were excised from mice of each of the therapeutic groups described above after 7 days from the end of three rounds of DC therapy to prepare effector cells. $^{51}$Cr release assay was performed using Yac-1 as the target. Further, to assay the cytotoxicity of T lymphocytes, the spleen cells remaining from the NK cell activity assay described above were cultured for 5 days with TRP-2 peptide, a B16 tumor antigen, to use them as effector cells. The effector cells were co-cultured with EL-4 target cells pulsed with mTRP-2 peptide, and then $^{51}$Cr release assay was performed. The rate of specific $^{51}$Cr release was calculated as follows:

$$[(\text{sample (cpm)} - \text{spontaneous emission (cpm)}) / (\text{maximum emission (cpm)} - \text{spontaneous emission (cpm)})] \times 100$$

where the maximum emission was determined using target cells incubated with 1% triton X, while spontaneous emission was determined using target cells incubated with culture medium alone.

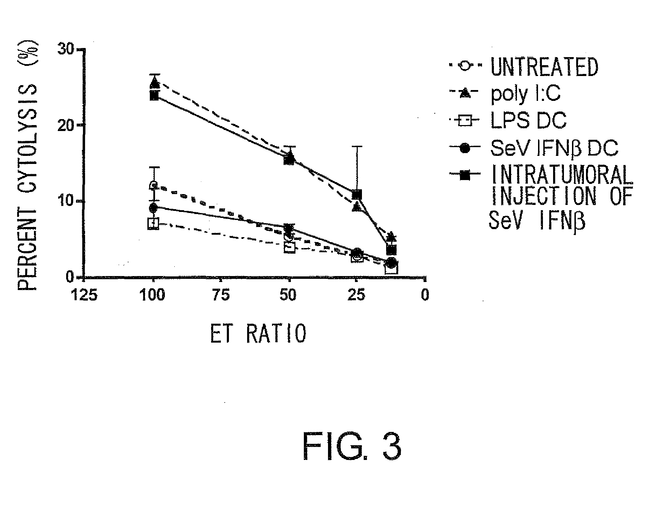

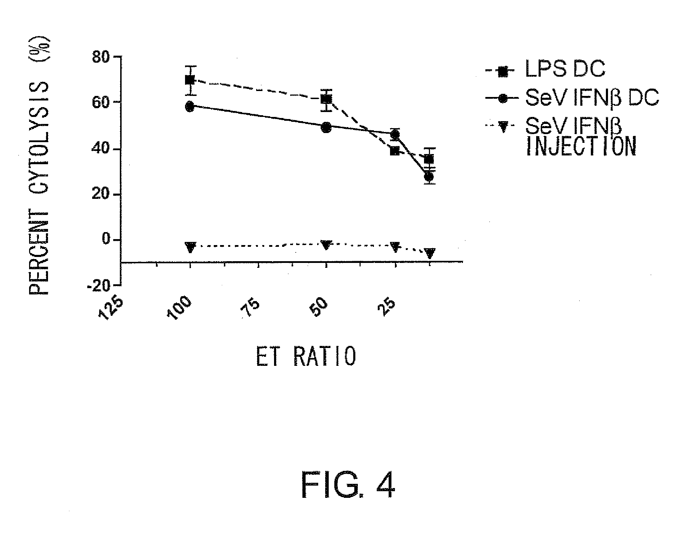

The activation of natural killer (NK) cells was detected only in mice that were directly injected with SeV, and not in the dendritic cell injection group (Fig. 3). In contrast, the activation of cytotoxic T lymphocytes (CTLs) was maximal in the DC/LPS treated group and mice treated with DC/SeV-IFNβ, slightly lower in the DC/SeV-GFP treated group, and was not detected in the group of SeV-IFNβ direct injection (Fig. 4). The tumor lysate pulsing had no significant influence on tumor growth nor on CTL response. Thus, it was revealed that direct administration of SeV-IFNβ strongly activates NK cells.

Therefore, the effect of tumor suppression by SeV administration was suggested to take place through a mechanism of NK cell activation. Furthermore, it was shown that an effective anticancer therapeutic effect is exhibited by both the administration of an SeV that does not carry a therapeutic gene and the administration of an SeV carrying a gene that is expected to be therapeutically effective.

Industrial Applicability

The present invention provides anticancer agents that contain a minus-strand RNA virus as an active ingredient. The RNA viruses of the present invention exhibit effective anticancer therapeutic effects without carrying any foreign gene that has therapeutic effects. Therefore, they may contribute greatly to the reduction of the cost for introducing foreign genes and to the reduction of the operational time for virus preparation. The present invention enables new virotherapies that use minus-strand RNA viruses.

**Claims**

1. An anticancer agent comprising a minus-strand RNA virus.

2. The anticancer agent of claim 1, wherein the minus-strand RNA virus does not encode a foreign protein.

3. The anticancer agent of claim 1 or 2, wherein the minus-strand RNA virus is an infectious or a non-infectious virion.

4. The anticancer agent of any one of claims 1 to 3, wherein the RNA virus is a genomic RNA-protein complex.

5. The anticancer agent of any one of claims 1 to 4, wherein the minus-strand RNA virus is a paramyxovirus.

6. The anticancer agent of claim 5, wherein the paramyxovirus is a Sendai virus.

7. A method for producing an anticancer agent, comprising the step of mixing a minus-strand RNA virus with a pharmaceutically acceptable carrier or medium.

8. A method for suppressing cancer, comprising the step of administering a minus-strand RNA virus to a cancer tissue.

FIG. 1

FIG. 2

FIG. 3

FIG. 4

## INTERNATIONAL SEARCH REPORT

| International application No. |
| --- |
| PCT/JP2005/008124 |

A. CLASSIFICATION OF SUBJECT MATTER
Int.Cl⁷ C12N5/10, A61K35/12, 48/00, A61P31/00, 35/00//C12N15/09

According to International Patent Classification (IPC) or to both national classification and IPC

B. FIELDS SEARCHED

Minimum documentation searched (classification system followed by classification symbols)
Int.Cl⁷ C12N5/10, A61K35/12, 48/00, A61P31/00, 35/00//C12N15/09

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)
BIOSIS/MEDLINE/WPIDS(STN), CA(STN), JSTPlus(JOIS)

C. DOCUMENTS CONSIDERED TO BE RELEVANT

| Category* | Citation of document, with indication, where appropriate, of the relevant passages | Relevant to claim No. |
| --- | --- | --- |
| X | Yasufumi KANEDA, Development of HVJ envelope vector for cancer therapy., Seikagaku (2003), Vol.75, No.8, page 737 | 1-7 |
| X Y | WO 2000/070070 A1 (DNAVEC Research Inc.), 23 November, 2000 (23.11.00), & US 2003-170266 A1 & US 2002/169306 A1 & WO 2003/025570 A1 & JP 2004-329222 A2 | 1,3-7 2 |
| X Y | JP 2002-272465 A (DNAVEC Research Inc.), 24 September, 2002 (24.09.02), & CA 2322057 A & US 6746860 B & US 2004/137627 A1 | 1,3-7 2 |

☒ Further documents are listed in the continuation of Box C.          ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 28 July, 2005 (28.07.05) | 16 August, 2005 (16.08.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/008124 |

C (Continuation). DOCUMENTS CONSIDERED TO BE RELEVANT

| Category* | Citation of document, with indication, where appropriate, of the relevant passages | Relevant to claim No. |
| --- | --- | --- |
| P,A | Hiraoka K et al., Enhanced tumor-specific long-term immunity of hemagglutinating [correction of hemaggluttinating] virus of Japan-mediated dendritic cell-tumor fused cell vaccination by coadministration with CpG oligodeoxynucleotides., J.Immunol. (2004 Oct), Vol.173, No.7, pages 4297 to 4307 | 1-7 |
| A | Jun KUNISAWA, Tadanori MAYUMI, "DDS Gijutsu o Mochiita Gan Chiryo -Maku Yugo Liposome no Gan Chiryo eno Oyo-", Jpn.J.Cancer Chemother (2001), Vol.28, No.5, pages 577 to 583 | 1-7 |
| A | Kanyama H et al., Usefulness of repeated direct intratumoral gene transfer using hemagglutinating virus of Japan-liposome method for cytosine deaminase suicide gene therapy., Cancer Res.(2001), Vol.61, No.1, pages 14 to 18 | 1-7 |

Form PCT/ISA/210 (continuation of second sheet) (January 2004)

## INTERNATIONAL SEARCH REPORT

| International application No. |
| --- |
| PCT/JP2005/008124 |

---

**Box No. II      Observations where certain claims were found unsearchable (Continuation of item 2 of first sheet)**

---

This international search report has not been established in respect of certain claims under Article 17(2)(a) for the following reasons:

1. ☒ Claims Nos.: 8
   because they relate to subject matter not required to be searched by this Authority, namely:
   The invention as set forth in claim 8 is relevant to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III      Observations where unity of invention is lacking (Continuation of item 3 of first sheet)**

---

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐   The additional search fees were accompanied by the applicant's protest.

☐   No protest accompanied the payment of additional search fees.

---

Form PCT/ISA/210 (continuation of first sheet (2)) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0070055 A
- WO 0070070 A
- WO 9716539 A
- WO 9716538 A
- WO 0118223 A

- WO 0104272 A, Curran, J.
- EP 1067179 A
- JP S6230752 B
- JP S6233879 B
- JP S6230753 B
- WO 9732010 A

**Non-patent literature cited in the description**

- **ALEMANY R. et al.** Replicative adenoviruses for cancer therapy. *Nat. Biotechnol.,* 2000, vol. 18, 723-727
- **CURIEL, D.T.** The development of conditionally replicative adenoviruses for cancer therapy. *Clin. Cancer Res.,* 2000, vol. 6, 3395-9
- **KIRN, D.** Virotherapy for cancer: Current status, hurdles, and future directions. *Cancer Gene Therapy,* 2002, vol. 9, 959-960
- **MINETA T. et al.** Attenuated multi-mutated herpes simplex virus-1 for the treatment of malignant gliomas. *Nat. Med.,* 1995, vol. 1, 938-943
- **ALEMANY R. et al.** Replicative adenoviruses for cancer therapy. *Nat Biotechnol.,* 2000, vol. 18, 723-727
- **CURIEL, D.T.** The development of conditionally replicative adenoviruses for cancer therapy. *Clin Cancer Res.,* 2000, vol. 6, 3395-9
- **KIRN, D.** Virotherapy for cancer: Current status, hurdles, and future directions. *Cancer Gene Therapy,* 2002, vol. 9, 959-960
- **MINETA T. et al.** Attenuated multi-mutated herpes simplex virus-1 for the treatment of malignant gliomas. *Nat Med,* 1995, vol. 1, 938-943
- **LI, H.-O. et al.** *J. Virol.,* 2000, vol. 74 (14), 6564-6569
- **INOUE, M. et al.** *J. Virol.,* 2003, vol. 77, 3238-3246
- **HENIKOFF, S. ; HENIKOFF, J. G.** *Proc. Natl. Acad. Sci. USA,* 1992, vol. 89, 10915-10919
- **MORIKAWA, Y. et al.** *Kitasato Arch. Exp. Med.,* 1991, vol. 64, 15-30
- **WRIGHT, K. E. et al.** *Virus Res.,* 2000, vol. 67, 49-57
- *J. Virology,* 1981, vol. 39, 519-528
- **HASAN, M. K. et al.** *J. Gen. Virol.,* 1997, vol. 78, 2813-2824

- **KATO, A. et al.** *EMBO J.,* 1997, vol. 16, 578-587
- **YU, D. et al.** *Genes Cells,* 1997, vol. 2, 457-466
- **DURBIN, A. P et al.** *Virology,* 1997, vol. 235, 323-332
- **WHELAN, S. P. et al.** *Proc. Natl. Acad. Sci. USA,* 1995, vol. 92, 8388-8392
- **SCHNELL. M. J. et al.** *EMBO J.,* 1994, vol. 13, 4195-4203
- **RADECKE, F. et al.** *EMBO J.,* 1995, vol. 14, 5773-5784
- **LAWSON, N. D. et al.** *Proc. Natl. Acad. Sci. USA,* vol. 92, 4477-4481
- **GARCIN, D. et al.** *EMBO J.,* 1995, vol. 14, 6087-6094
- **KATO, A. et al.** *Genes Cells,* 1996, vol. 1, 569-579
- **BARON, M. D. ; BARRETT, T.** *J. Virol.,* 1997, vol. 71, 1265-1271
- **BRIDGEN, A. ; ELLIOTT, R. M.** *Proc. Natl. Acad. Sci. USA,* 1996, vol. 93, 15400-15404
- **BAFFOUR, R. et al.** *J. Vasc. Surg.,* 1992, vol. 16 (2), 181-91
- **PU, L. Q. et al.** *J. Surg. Res.,* 1993, vol. 54 (6), 575-83
- **TAKESHITA, S. et al.** *Circulation,* 1994, vol. 90 (5 Pt 2), II228-34
- **TAKESHITA, S. et al.** *J. Clin, Invest.,* 1994, vol. 93 (2), 662-70
- **A. HANNEKEN ; A. BAIRD.** *Investigative Ophthalmology & Visual Science,* 1995, vol. 36, 1192-1196
- **TAKAISHI, S. et al.** *Biochem. Biophys. Res. Commun.,* 2000, vol. 267 (2), 658-62
- **SENO M et al.** *Cytokine,* 1998, vol. 10 (4), 290-4

- **HANNEKEN, A.** *FEBS Lett.,* 2001, vol. 489, 176
- **ARAI et al.** *J. Immunol.,* 1989, vol. 142 (1), 274-282
- **YASUKAWA et al.** *EMBO J.,* 1987, vol. 6 (10), 2939-2945
- **WOLF et al.** *J. Immunol.,* 1991, vol. 146 (9), 3074-3081
- **GREN et al.** *J. Interferon Res.,* 1984, vol. 4 (4), 609-617
- **WEISMANN et al.** *Princess Takamatsu Symp.,* 1982, vol. 12, 1-22
- **DERYNCK, R. et al.** *Nature,* 1980, vol. 285, 542-547
- **HIGASHI, Y et al.** *J. Bio,. Chem.,* 1983, vol. 258, 9522-9529
- **KUGA, T. et al.** *Nucleic Acid Res.,* 1989, vol. 17, 3291
- **PENNICA et al.** *Nature,* 1984, vol. 312, 724-729
- **HIRANO et al.** *Nature,* 1986, vol. 324, 73-76
- **CANTRELL et al.** *Proc. Natl. Acad. Sci.,* 1985, vol. 82 (18), 6250-6254
- **Y. NAGAI ; A. KATO.** *Microbiol. Immunol.,* 1999, vol. 43, 613-624
- **NAVIAUX, R. K. et al.** *J. Virol.,* 1996, vol. 70, 5701-5705
- **KATO, A. et al.** *J. Virol.,* 1997, vol. 71, 7266-7272
- Paramyxoviridae: The viruses and their replication. **LAMB, R.A. ; KOLAKOFSKY, D.** Fields of Virology. Lippincott - Raven Publishers, 1996, vol. 2, 1177-1204
- **HURWITZ, J.L. et al.** *Vaccine,* 1997, vol. 15, 533-540
- *Journal of Virology,* 1993, vol. 67 (8), 4822-4830
- **HASAN, M. K. et al.** *J. Gen. Virol.,* 1997, vol. 78, 2813-2820
- **KOLAKOFSKI, D. et al.** *J. Virol.,* 1998, vol. 72, 891-899
- **CALAIN, P. ; ROUX, L.** *J. Virol.,* 1993, vol. 67, 4822-4830
- **HASAN, M. K. et al.** *J. General Virology,* 1997, vol. 78, 2813-2820
- **DURBIN, A. P. et al.** *Virology,* 1997, vol. 235, 323-332
- **GRAHAM, F. L. ; VAN DER EB, J.** *Virology,* 1973, vol. 52, 456
- **WIGLER, M. ; SILVERSTEIN, S.** *Cell,* 1977, vol. 11, 223
- **CHEN, C. ; OKAYAMA, H.** *Mol. Cell. Biol.,* 1987, vol. 7, 2745
- **CALOS, M. P.** *Proc. Natl. Acad. Sci. USA,* 1983, vol. 80, 3015
- **FUERST, T. R. et al.** *Proc. Natl. Acad. Sci. USA,* 1986, vol. 83, 8122-8126
- Hemaggulutinating virus of Japan-liposome-mediated gene delivery to vascular cells. **YONEMITSU, Y. ; KANEDA, Y.** Molecular Biology of Vascular Diseases. Method in Molecular Medicine. Humana Press, 1999, 295-306
- *State-of-the-Art Technology Protocol in Neuroscience Research III, Molecular Neuron Physiology,* 1993, 153-172
- **TASHIRO, M.** Virus Experiment Protocol. Medical View Co., Ltd, 1995, 68-73
- **ARAI, T. et al.** *J. Virology,* 1998, vol. 72, 1115-1121
- **SAITO et al.** *Nucl. Acids Res.,* 1995, vol. 23, 3816-3821
- **ARAI, T. et al.** *J. Virol,* 1998, vol. 72, 1115-1121
- **FUERST, T.R. et al.** *Proc. Natl. Acad. Sci. USA,* 1986, vol. 83, 8122-8126
- Uirusu Jikken-gaku kakuron. Maruzen, 1982
- **ITOH H. et al.** *Jap. J. Med. Sci. Biol.,* 1970, vol. 23, 227
- **MATSUMOTO M.** *Bact. Rev.,* 1966, vol. 30, 152
- **SEDWIK W. D.** *J. Virol.,* 1967, vol. 1, 1224
- **LAVER W. G.** *Fundamental Techniques in Virology,* 1969, vol. 82
- **KIYOTANI, K. et al.** *Virology,* 1990, vol. 177 (1), 65-74